(19) Europäisches Patentamt / European Patent Office / Office européen des brevets

(11) **EP 3 998 253 A1**

(12) **EUROPEAN PATENT APPLICATION**
published in accordance with Art. 153(4) EPC

(43) Date of publication:
**18.05.2022 Bulletin 2022/20**

(21) Application number: **20839572.3**

(22) Date of filing: **02.07.2020**

(51) International Patent Classification (IPC):
**C07C 407/00** (2006.01)   **C07C 17/278** (2006.01)
**C07C 19/16** (2006.01)   **C07C 409/34** (2006.01)

(52) Cooperative Patent Classification (CPC):
(C-Sets available)
**C07C 17/278; C07C 407/00**   (Cont.)

(86) International application number:
**PCT/JP2020/026079**

(87) International publication number:
**WO 2021/010183 (21.01.2021 Gazette 2021/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.07.2019   JP 2019130510**

(71) Applicant: **AGC INC.**
**Chiyoda-ku,**
**Tokyo 1008405 (JP)**

(72) Inventors:
• **FUJITA, Tomoyuki**
  **Tokyo 100-8405 (JP)**
• **FUKUNAGA, Shintaro**
  **Tokyo 100-8405 (JP)**

(74) Representative: **Müller-Boré & Partner**
**Patentanwälte PartG mbB**
**Friedenheimer Brücke 21**
**80639 München (DE)**

(54) **PERFLUOROACYL PEROXIDE PRODUCTION METHOD, AND FLUOROALKYL IODIDE PRODUCTION METHOD**

(57)   To provide a method for producing a perfluoroacyl peroxide which can suppress formation of solid matters in a reaction apparatus, and a method for producing a fluoroalkyl iodide.

The method for producing a perfluoroacyl peroxide of the present invention is a method for producing a perfluoroacyl peroxide, which comprises:
mixing an organic solvent solution containing a perfluoroacyl halide and an organic solvent which is at least one perfluoroalkyl iodide selected from the group consisting of $C_2F_5I$, $C_4F_9I$ and $C_6F_{13}I$,
a first aqueous solution containing hydrogen peroxide, and
a second aqueous solution containing a basic alkali metal compound,
to obtain a mixed liquid, and
reacting the perfluoroacyl halide and the hydrogen peroxide in the mixed liquid to produce a perfluoroacyl peroxide,
wherein the pH of the aqueous phase of the mixed liquid is adjusted to be from 7 to 14.

Fig. 1

**(Cont. next page)**

(52) Cooperative Patent Classification (CPC): (Cont.)

C-Sets
**C07C 17/278, C07C 19/16;**
**C07C 407/00, C07C 409/34**

**Description**

TECHNICAL FIELD

The present invention relates to a method for producing a perfluoroacyl peroxide and a method for producing a fluoroalkyl iodide.

BACKGROUND ART

As a method for producing a perfluoroacyl peroxide, a method of mixing a solution containing a perfluoroacyl halide and dichloropentafluoropropane ($CClF_2CF_2CHClF$) as an organic solvent, an aqueous hydrogen peroxide solution, and an aqueous potassium hydroxide solution, for reaction has been known (Patent Document 1).

PRIOR ART DOCUMENTS

PATENT DOCUMENT

Patent Document 1: WO2010/117029

DISCLOSURE OF INVENTION

TECHNICAL PROBLEM

Under restrictions on use of substances of concern in recent years, in production of perfluoroacyl peroxides, organic solvents which replace dichloropentafluoropropane have been desired.
The present inventors have found that perfluoroacyl peroxides can be produced by using $C_2F_5I$, $C_4F_9I$ or $C_6F_{13}I$ as an organic solvent which replaces dichloropentafluoropropane. However, they have found that when $C_2F_5I$, $C_4F_9I$ or $C_6F_{13}I$ is used for producing perfluoroacyl peroxides, solid matters containing iodine derived from $C_2F_5I$, $C_4F_9I$ or $C_6F_{13}I$ form in a reaction apparatus. The solid matters formed in the reaction apparatus may clog the flow path.
Under these circumstances, it is an object of the present invention to provide a method for producing a perfluoroacyl peroxide which can suppress formation of solid matters in the reaction apparatus, and a method for producing a fluoroalkyl iodide.

SOLUTION TO PROBLEM

The present inventors have conducted extensive studies on the above object and as a result, found that in a case where $C_2F_5I$, $C_4F_9I$ or $C_6F_{13}I$ is used as an organic solvent in production of a perfluoroacyl peroxide, by adjusting the pH of the aqueous phase of the mixed liquid of solutions used for the reaction to be within a predetermined range, formation of solid matters in the reaction apparatus can be suppressed, and they have accomplished the present invention.
That is, the present inventors have found that the above object can be achieved by the following constitutions.

[1] A method for producing a perfluoroacyl peroxide, which comprises:

mixing an organic solvent solution containing a perfluoroacyl halide and an organic solvent which is at least one perfluoroalkyl iodide selected from the group consisting of $C_2F_5I$, $C_4F_9I$ and $C_6F_{13}I$,
a first aqueous solution containing hydrogen peroxide, and
a second aqueous solution containing a basic alkali metal compound, to obtain a mixed liquid, and
reacting the perfluoroacyl halide and the hydrogen peroxide in the mixed liquid to produce a perfluoroacyl peroxide,
wherein the pH of the aqueous phase of the mixed liquid is adjusted to be from 7.0 to 14.0.

[2] The method for producing a perfluoroacyl peroxide according to [1], wherein the first aqueous solution and the second aqueous solution are mixed to obtain a mixed aqueous solution, and the mixed aqueous solution and the organic solvent solution are mixed in a reactor.
[3] The method for producing a perfluoroacyl peroxide according to [2], wherein a wetted part of the reactor is made of silicon carbide.
[4] The method for producing a perfluoroacyl peroxide according to any one of [1] to [3], wherein the perfluoroacyl halide is a compound represented by the following formula (1):

$$G(CF_2)_v[CF(CF_3)CF_2]_w[OCF(CF_3)CF_2]_y[OCF(CF_3)]_z\text{-}(C=O)X \qquad (1)$$

wherein G is a fluorine atom or a pentafluorophenoxy group, X is a halogen atom, v is an integer of from 0 to 10, w is 0 or 1, y is an integer of from 0 to 7, and z is 0 or 1, provided that $v+w \geqq 1$ is satisfied.

[5] The method for producing a perfluoroacyl peroxide according to any one of [1] to [4], wherein the first aqueous solution is an aqueous hydrogen peroxide solution.

[6] The method for producing a perfluoroacyl peroxide according to any one of [1] to [4], wherein the first aqueous solution contains a metal peroxide.

[7] The method for producing a perfluoroacyl peroxide according to any one of [1] to [6], wherein the basic alkali metal compound is potassium hydroxide.

[8] The method for producing a perfluoroacyl peroxide according to any one of [1] to [7], wherein the content of the perfluoroacyl halide in the organic solvent solution to the total mass of the organic solvent solution is from 1 to 80 mass%.

[9] The method for producing a perfluoroacyl peroxide according to any one of [1] to [8], wherein the perfluoroacyl halide and the hydrogen peroxide are reacted at from -30 to 50°C.

[10] The method for producing a perfluoroacyl peroxide according to any one of [1] to [9], wherein the organic solvent solution and the first aqueous solution are mixed so that the molar ratio of the hydrogen peroxide to the perfluoroacyl halide is from 1.0 to 3.0.

[11] The method for producing a perfluoroacyl peroxide according to any one of [1] to [10], wherein the organic solvent solution and the second aqueous solution are mixed so that the molar ratio of the basic alkali metal compound to the perfluoroacyl halide is from 1.0 to 2.0.

[12] The method for producing a perfluoroacyl peroxide according to any one of [1] to [11], wherein the organic solvent solution and the second aqueous solution are mixed so that the molar ratio of the basic alkali metal compound to the perfluoroalkyl iodide is from 1.0 to 2.2.

[13] The method for producing a perfluoroacyl peroxide according to any one of [1] to [12], wherein the first aqueous solution and the second aqueous solution are mixed so that the molar ratio of the basic alkali metal compound to the hydrogen peroxide is from 0.5 to 1.5.

[14] A method for producing a fluoroalkyl iodide, which comprises reacting a fluoroalkyl iodide represented by the following formula (3) and tetrafluoroethylene in the presence of a perfluoroacyl peroxide obtained by the method for producing a perfluoroacyl peroxide as defined in any one of [1] to [13], to obtain a fluoroalkyl iodide represented by the following formula (4):

$$R^f I \qquad (3)$$

$$R^f CF_2 CF_2 I \qquad (4)$$

wherein $R^f$ is a $C_{1\text{-}4}$ fluoroalkyl group.

ADVANTAGEOUS EFFECTS OF INVENTION

According to the present invention, it is possible to provide a method for producing a perfluoroacyl peroxide which can suppress formation of solid matters in a reaction apparatus, and a method for producing a fluoroalkyl iodide.

BRIEF DESCRIPTION OF DRAWINGS

Fig. 1 is a schematic view illustrating an example of a reaction apparatus to conduct the method for producing a perfluoroacyl peroxide of the present invention.
Fig. 2 is a schematic view partially illustrating an example of a reaction apparatus to conduct the method for producing a perfluoroacyl peroxide of the present invention.

DESCRIPTION OF EMBODIMENTS

[Method for producing perfluoroacyl peroxide]

The method for producing a perfluoroacyl peroxide of the present invention is a method for producing a perfluoroacyl peroxide, which comprises:

> mixing an organic solvent solution containing a perfluoroacyl halide and an organic solvent which is at least one perfluoroalkyl iodide selected from the group consisting of $C_2F_5I$, $C_4F_9I$ and $C_6F_{13}I$,
> a first aqueous solution containing hydrogen peroxide, and
> a second aqueous solution containing a basic alkali metal compound, to obtain a mixed liquid, and
> reacting the perfluoroacyl halide and the hydrogen peroxide in the mixed liquid to produce a perfluoroacyl peroxide.

In the method for producing a perfluoroacyl peroxide of the present invention, the pH of the aqueous phase of the mixed liquid is adjusted to be from 7.0 to 14.0.

According to the method for producing a perfluoroacyl peroxide of the present invention, formation of solid matters in a reaction apparatus can be suppressed. The details of the reason are not clearly understood yet, but the following reasons are estimated. That is, when the compounds contained in the mixed liquid are reacted, solid matters containing iodine derived from the perfluoroalkyl iodide may form, but by adjusting the pH of the aqueous phase of the mixed liquid to be within the above range, the solid matters containing iodine are dissolved, and formation of the solid matters can be suppressed. Thus, clogging of the flow path in the reaction apparatus can also be suppressed.

<Organic solvent solution>

The perfluoroacyl halide contained in the organic solvent solution is preferably a compound represented by the following formula (1), whereby the perfluoroacyl peroxide will be readily produced, and the raw material will be readily prepared.

$$G(CF_2)_v[CF(CF_3)CF_2]_w[OCF(CF_3)CF_2]_y[OCF(CF_3)]_z\text{-}(C=O)X \qquad (1)$$

wherein G is a fluorine atom or a pentafluorophenoxy group, X is a halogen atom, v is an integer of from 0 to 10, w is 0 or 1, y is an integer of from 0 to 7, and z is 0 or 1, provided that $v+w \geqq 1$ is satisfied.

G is preferably a fluorine atom, and X is preferably a fluorine atom or a chlorine atom. v is preferably an integer of from 1 to 5, and y is preferably an integer of from 0 to 2. Particularly, it is preferred that G is a fluorine atom, X is a fluorine atom or a chlorine atom, v is 2 or 4, and w+y+z is 0.

As specific examples of the perfluoroacyl halide, perfluoroethanoyl chloride, perfluoroethanoyl fluoride, perfluoropropanoyl chloride, perfluoropropanoyl fluoride, perfluorobutanoyl chloride, perfluorobutanoyl fluoride, perfluoropentanoyl chloride, perfluoropentanoyl fluoride, perfluorohexanoyl chloride, perfluorohexanoyl fluoride, perfluoro-2,5-dimethyl-3,6-dioxanonanoyl fluoride, perfluoro-2,5,8-trimethyl-3,6,9-trioxadodecanoyl fluoride, and perfluoro-2-methyl-3-oxahexanoyl fluoride may be mentioned. Among them, in view of availability and easy production, perfluoropropanoyl chloride, perfluoropropanoyl fluoride, perfluorobutanoyl chloride and perfluorobutanoyl fluoride are preferred, and perfluoropropanoyl chloride is particularly preferred. The perfluoroacyl halide may be used in combination of two or more types.

The organic solvent solution contains, as the organic solvent, at least one perfluoroalkyl iodide selected from the group consisting of $C_2F_5I$, $C_4F_9I$ and $C_6F_{13}I$.

As the organic solvent contained in the organic solvent solution, only one type of the perfluoroalkyl iodide may be used, or two or more types may be used in combination. Each of the above perfluoroalkyl iodides does not react with the peroxide and is excellent in solubility of the perfluoroacyl halide and the perfluoroacyl peroxide.

The organic solvent solution may contain a component other than the perfluoroacyl halide and the perfluoroalkyl iodide. Such other component may be an organic solvent other than the perfluoroalkyl iodide.

As other component contained in the organic solvent solution, preferred are components which do not react with the peroxide, such as hydrofluorocarbons (such as $CF_3CF_2CF_2CF_2H$), hydrofluoroethers (such as $CF_3CH_2OCF_2CF_2H$) and hydrolysates of the perfluoroacyl halides (such as perfluoroethanoic acid).

The content of the perfluoroacyl halide in the organic solvent solution to the total mass of the organic solvent solution is preferably from 1 to 80 mass%, more preferably form 3 to 70 mass%, particularly preferably from 10 to 60 mass%. When it is at most the upper limit, self-decomposition of the obtainable perfluoroacyl peroxide tends to be suppressed. When it is at least the lower limit, the perfluoroacyl peroxide will be obtained with more excellent productivity, and such is industrially preferred.

The content of the perfluoroalkyl iodide in the organic solvent solution to the total mass of the organic solvent solution is preferably from 20 to 99 mass%, more preferably from 30 to 97 mass%, further preferably from 40 to 97 mass%, particularly preferably from 40 to 90 mass%.

When the organic solvent solution contains a component other than the perfluoroacyl halide and the perfluoroalkyl iodide, its content to the total mass of the organic solvent solution is preferably at most 10 mass%, more preferably at most 2 mass%, further preferably at most 1 mass%.

The total content of the perfluoroacyl halide and the perfluoroalkyl iodide in the organic solvent solution is preferably at least 90 mass%, more preferably at least 98 to 100 mass%.

<First aqueous solution>

The first aqueous solution is an aqueous solution containing hydrogen peroxide, and may be an aqueous solution obtained by dissolving a compound capable of generating hydrogen peroxide upon reaction with water thereby containing hydrogen peroxide, and an aqueous hydrogen peroxide solution. The compound capable of generating hydrogen peroxide upon reaction with water is preferably a metal peroxide. By dissolving the metal peroxide in water, an aqueous solution containing hydrogen peroxide, a metal hydroxide and an unreacted metal peroxide can be obtained.

The first aqueous solution is preferably an aqueous hydrogen peroxide solution, in view of handling efficiency and economical efficiency. In a case where an aqueous hydrogen peroxide solution is used as the first aqueous solution, by mixing it with the after-described second aqueous solution, an alkali metal peroxide forms in the mixed liquid.

As specific examples of the metal peroxide, sodium peroxide, barium peroxide and potassium peroxide may be mentioned. In that the reaction apparatus is hardly clogged, the metal peroxide is preferably sodium peroxide or potassium peroxide. When the metal peroxide is used, two or more types may be used in combination.

In preparation of the first aqueous solution, it is preferred to use either one of hydrogen peroxide and the metal peroxide alone without being combined.

In a case where the first aqueous solution is the aqueous hydrogen peroxide solution, the content of hydrogen peroxide in the first aqueous solution to the total mass of the first aqueous solution is preferably from 1 to 60 mass%, more preferably from 5 to 50 mass%, particularly preferably from 10 to 40 mass%. When it is at most the upper limit, the perfluoroacyl peroxide will be obtained with excellent yield. When it is at least the lower limit, the perfluoroacyl peroxide will be obtained with excellent productivity, and such is industrially preferred.

In a case where the first aqueous solution is the aqueous hydrogen peroxide solution, as a component other than hydrogen peroxide contained in the first aqueous solution, a component which does not react with the peroxide is preferred, and for example, an alkali metal salt such as sodium chloride or potassium chloride may be mentioned. In a case where the first aqueous solution contains a component other than hydrogen peroxide, its content is preferably at most 10 mass%, more preferably at most 5 mass%, further preferably at most 1 mass%.

Further, in a case where the first aqueous solution is an aqueous solution containing hydrogen peroxide, a metal hydroxide and an unreacted metal peroxide, the total of the amount of the hydrogen peroxide contained in the aqueous solution and the amount of hydrogen peroxide which may be generated from the unreacted metal peroxide in the aqueous solution (or, in a case where the unreacted metal peroxide directly contributes to the reaction, the amount corresponding to the hydrogen peroxide) can be considered to be equivalent to the content of hydrogen peroxide in the first aqueous solution in a case where the first aqueous solution is the aqueous hydrogen peroxide solution.

<Second aqueous solution>

The basic alkali metal compound contained in the second aqueous solution is an alkali metal compound of which the aqueous solution is basic, and is preferably a hydroxide, carbonate, and hydrogencarbonate of an alkali metal.

As specific examples of the basic alkali metal compound, sodium hydroxide, potassium hydroxide, sodium hydrogencarbonate, potassium hydrogencarbonate, sodium carbonate and potassium carbonate may be mentioned. Among them, potassium hydroxide is preferred, which is highly soluble and provides favorable productivity of the perfluoroacyl peroxide.

The basic alkali metal compound may be used in combination of two or more types.

Further, the second aqueous solution may further contain a component other than the basic alkali metal compound.

The component other than the basic alkali metal compound contained in the second aqueous solution is preferably a component which does not react with the basic alkali metal compound and the peroxide, and may, for example, be an alkali metal salt such as sodium chloride or potassium chloride.

The content of the basic alkali metal compound in the second aqueous solution to the total mass of the second aqueous solution is preferably from 1 to 60 mass%, more preferably from 3 to 50 mass%, particularly preferably from 5 to 30 mass%. When it is at most the upper limit, the perfluoroacyl peroxide will be obtained with excellent yield. When it is at least the lower limit, the perfluoroacyl peroxide will be obtained with more excellent productivity, and such is industrially preferred.

In a case where the second aqueous solution contains a component other than the basic alkali metal compound, its content is preferably at most 10 mass%, more preferably at most 5 mass%, further preferably at most 1 mass%.

<Production process>

The method for producing a perfluoroacyl peroxide of the present invention may be conducted either by continuous process or by batch process, but is preferably conducted by continuous process. By continuous process, as the amount of the raw materials and the product remaining in the reactor is small, safe production with high yield is possible in a case where the reaction of forming the product from the raw materials is an exothermic reaction, or in a case where the product is thermally unstable and decomposes accompanied by heat generation. Further, by the reaction apparatus in the continuous process, the production amount is increased as compared with the reaction apparatus of a size at the same level in the batch process.

In a case where the organic solvent solution, the first aqueous solution and the second aqueous solution are mixed to obtain a mixed liquid, the order of mixing these three solutions is not particularly limited, and the three solutions may be mixed at the same time to obtain a mixed liquid, or two solutions may be mixed and the other one solution is mixed with the mixture to obtain a mixed liquid. As the mixing order, preferred is a method of supplying the three solutions all at once to the reactor and mixing them in the reactor to obtain a mixed liquid, or a method of supplying a mixed aqueous solution having the first aqueous solution and the second aqueous solution preliminarily mixed, and the organic solvent solution, to the reactor, and mixing these two solutions in the reactor to obtain a mixed liquid, and the latter method of supplying the mixed aqueous solution and the organic solvent solution to the reactor to obtain a mixed liquid is particularly preferred, whereby good yield tends to be obtained.

An example of the method for producing a perfluoroacyl peroxide of the present invention will be described with reference to drawings.

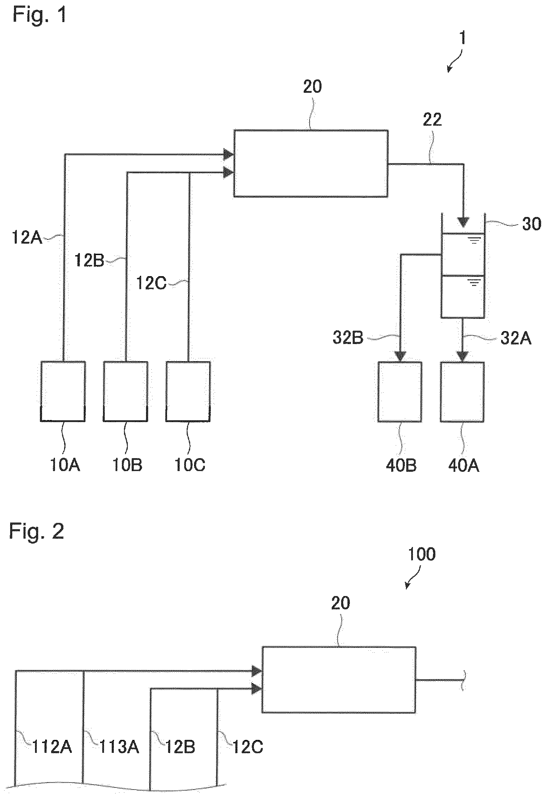

Fig. 1 is a schematic view illustrating an example of a reaction apparatus to conduct the method for producing a perfluoroacyl peroxide of the present invention.

The reaction apparatus 1 has a storage tank 10A capable of storing the above organic solvent solution, a storage tank 10B capable of storing the above first aqueous solution, a storage tank 10C capable of storing the above second aqueous solution, a reactor 20, a two phase separation tank 30, an organic phase recovery tank 40A, and an aqueous phase recovery tank 40B.

The storage tank 10A, the storage tank 10B and the storage tank 10C are tanks to store the respective solutions to be supplied to the reactor 20. The storage tank 10A, the storage tank 10B and the storage tank 10C are connected to the reactor 20 via a supply tube 12A, a supply tube 12B and a supply tube 12C so as to supply the respective solutions to the reactor 20. The supply tube 12B and the supply tube 12C are merged on the upstream side of the reactor 20, whereby a solution having the first aqueous solution and the second aqueous solution mixed (hereinafter sometimes referred to as mixed aqueous solution) is supplied to the reactor 20.

The supply tube 12A, the supply tube 12B and the supply tube 12C may have a flow rate controlling mechanism (for example, a control valve) which is not shown, to control the flow rate of each solution.

In Fig. 1, the supply tube 12B and the supply tube 12C are merged on the upstream side of the reactor 20, however, the reaction apparatus is not limited to such a structure, and the respective supply tubes 12B and 12C may directly be connected to the reactor 20 without the supply tubes being merged.

Fig. 1 illustrates the reaction apparatus 1 in which the organic solvent solution stored in the storage tank 10A is supplied to the reactor 20, however, the reaction apparatus is not limited to such a structure, and a reaction apparatus 100 shown in Fig. 2 may be used.

Fig. 2 is a schematic view partially illustrating an example of a reaction apparatus to conduct the method for producing a perfluoroacyl peroxide of the present invention.

The reaction apparatus 100 shown in Fig. 2 has no storage tank 10A, has a supply tube 112A capable of supplying the perfluoroacyl halide, a supply tube 113A capable of supplying the organic solvent which is the perfluoroalkyl iodide, and has a structure such that a supply tube 112A and a supply tube 113A are merged on the upstream side of the reactor 20 so that the organic solvent solution containing the perfluoroacyl halide and the organic solvent is introduced to the reactor 20. Although not shown in Fig. 2, the reaction apparatus 100 in Fig. 2 may have a storage tank which stores the perfluoroacyl halide and a storage tank which stores the organic solvent. The other structure is the same as that of the reaction apparatus in Fig. 1, and the description is omitted.

The reactor 20 is used to mix the solutions supplied to the reactor 20 and to react the compounds contained in the obtained mixed liquid. The reactor 20 is connected to the two phase separation tank 30 via the supply tube 22 so that a liquid containing the perfluoroacyl peroxide produced in the reactor 20 is supplied to the two phase separation tank 30.

The reactor 20 may be either reactor of continuous process or batch process, and as described above, a reactor of continuous process is preferred. The reactor of continuous process is preferably a tubular reactor, which has a large heat transfer area.

The reactor 20 preferably has a structure capable of heating and cooling at the jacket portion and has a structure such that the solutions can be continuously added uniformly.

The reactor 20 may have a thermometer to control the internal temperature of the reactor 20 or a pH meter to control the pH of the aqueous phase of the mixed liquid.

Fig. 1 illustrates an example of the reaction apparatus having one reactor 20, however, a reaction apparatus having a plurality of reactors arranged in parallel may be used. A reaction apparatus having a plurality of reactors is preferred in that the amount of the perfluoroacyl peroxide produced can properly be controlled.

As the reactor, a known one may be used, for example, a reactor having properties such as the shape, the tube length and the cross sectional area as described in WO2010/117029 may be used.

In order that the organic solvent solution and the mixed aqueous solution (or, the first aqueous solution and the second aqueous solution) which are immiscible with each other are uniformly and finely dispersed and mixed, inside of the reactor 20 preferably has a mixing region. This mixing region is a region having e.g. a static mixing device, a filling, an ultrasonic mixing device, a mechanical mixing device or the like provided in the flow path in the reactor 20.

Particularly, it is preferred to provide a static mixing device in the flow path to mix the organic solvent solution, and the first aqueous solution and the second aqueous solution, which are immiscible, with dividing, inverting or converting them.

As specific examples of the static mixing device, static mixers (such as Stator-tube mixer type and spiral mixer type) may be mentioned. As specific examples of the filling, fillings having a diameter smaller than the inner diameter of a capillary forming the flow path (such as resin pellets, Raschig rings, Lessing rings, pall rings, saddles and Sulzer packing) may be mentioned.

As a material of the wetted part of the reactor 20, a material other than a metal is preferred, in order to suppress decomposition of the peroxide by a metal and metal ions. For example, a hydrocarbon resin such as polyethylene, a fluororesin such as polytetrafluoroethylene or a tetrafluoroethylene/perfluoroalkyl vinyl ether copolymer (hereinafter sometimes referred to as "PFA"), glass or silicon carbide may be mentioned, and silicon carbide is preferred in that it is excellent in thermal conductivity and thereby provides excellent cooling efficiency when the reactor is cooled, the internal temperature can readily be maintained low thereby to suppress decomposition of the peroxide as the product and the solvent, and solid matters are less likely to form in the reactor. The wetted part means a portion which may be in contact with the solutions introduced into the reactor 20 and the mixed liquid of the solutions introduced.

The two phase separation tank 30 is used to separate the mixed liquid containing the perfluoroacyl peroxide as the reaction product (hereinafter sometimes referred to as "mixed liquid after the reaction") into an aqueous phase and an organic phase. For example, the mixed liquid after the reaction may be separated into an aqueous phase and an organic phase utilizing the difference in the specific gravity. The formed perfluoroacyl peroxide is contained in the organic phase.

The two phase separation tank 30 is connected to the organic phase recovery tank 40A via a supply tube 32A and is connected to the aqueous phase recovery tank 40B via a supply tube 32B so that the organic phase and the aqueous phase separated are recovered.

The method for producing a perfluoroacyl peroxide using the reaction apparatus 1 shown in Fig. 1 will be described in detail.

First, the organic solvent solution stored in the storage tank 10A, the first aqueous solution stored in the storage tank 10B and the second aqueous solution stored in the storage tank 10C are supplied via the supply tube 12A to the supply tube 12C so that the organic solvent solution and the mixed aqueous solution are introduced to the reactor 20 at predetermined flow rates. The solutions are mixed in the reactor 20 to obtain a mixed liquid containing the organic solvent solution and the mixed aqueous solution. The flow rate of the mixed aqueous solution is the total of the flow rate of the first aqueous solution and the flow rate of the second aqueous solution.

Since the organic solvent solution and the mixed aqueous solution are immiscible, the reaction to form the perfluoroacyl peroxide proceeds at the interface between the organic solvent solution and the aqueous solution. Accordingly, it is preferred to more uniformly and finely disperse and mix the organic solvent solution, and the first aqueous solution and the second aqueous solution.

Then, the mixed liquid after the reaction discharged from the reactor 20 is introduced to the two phase separation tank 30 via the supply tube 22. The mixed liquid after the reaction which contains the organic solvent solution and the mixed aqueous solution which are immiscible with each other, is separated into an organic phase and an aqueous phase in the two phase separation tank 30.

And, the two liquids separated in the two phase separation tank 30, that is the organic phase and the aqueous phase are recovered in the organic phase recovery tank 40A and in the aqueous phase recovery tank 40B respectively via the supply tube 32A and the supply tube 32B. As described above, the formed perfluoroacyl peroxide is contained in the organic phase, and thus is recovered in the organic phase recovery tank 40A.

The pH of the aqueous phase of the mixed liquid is adjusted to be from 7.0 to 14.0. With a view to further improving the solubility of the solid matters, the pH of the aqueous phase of the mixed liquid is adjusted to be preferably at least 7.2, particularly preferably at least 7.4, and in order that the perfluoroacyl peroxide will be obtained with more excellent yield, preferably at most 13.5, particularly preferably at most 13.0.

As specific examples of the method to adjust the pH of the aqueous phase of the mixed liquid, a method of adjusting the flow rates of the organic solvent solution, the first aqueous solution and the second aqueous solution, and a method of adjusting the contents of the components contained in the solutions may be mentioned.

The pH of the aqueous phase of the mixed liquid means the pH at $25\pm2°C$, and may be measured by a known pH meter.

The flow rate ratio of the organic solvent solution and the first aqueous solution to be introduced to the reactor 20 is adjusted so that the molar ratio of hydrogen peroxide to the total amount of the perfluoroacyl halide in the respective solutions (hydrogen peroxide/perfluoroacyl halide) is preferably within a range of from 1.0 to 3.0, more preferably from 1.1 to 2.5, particularly preferably from 1.2 to 2.3. When the molar ratio is within the above range, the pH of the aqueous phase of the mixed liquid will readily be adjusted to be within the above range. Further, the flow rate ratio is adjusted so that the molar ratio of hydrogen peroxide to the perfluoroalkyl iodide in the respective solutions ([hydrogen peroxide]/[perfluoroalkyl iodide]) is preferably within a range of from 1.0 to 2.2, more preferably from 1.05 to 2.15, particularly preferably from 1.10 to 2.10. When the molar ratio is within the above range, the pH of the aqueous phase of the mixed liquid will readily be adjusted to be within the above range.

The flow rate ratio of the organic solvent solution and the second aqueous solution (the second aqueous solution before mixed into the mixed aqueous solution) to be introduced to the reactor 20 is adjusted so that the molar ratio of the basic alkali metal compound to the total amount of the perfluoroacyl halide in the respective solutions (basic alkali metal compound/total amount of perfluoroacyl halide) is preferably within a range of from 1.0 to 2.0, more preferably from 1.05 to 1.90, particularly preferably from 1.10 to 1.70. When the molar ratio is within the above range, the pH of the aqueous phase of the mixed liquid will readily be adjusted to be within the above range.

Further, the flow rate ratio is adjusted so that the total molar ratio of the basic alkali metal compound to the perfluoroalkyl iodide in the respective solutions (total amount of basic alkali metal compound / perfluoroalkyl iodide) is preferably within a range of from 1.0 to 2.2, more preferably from 1.05 to 2.10, particularly preferably from 1.10 to 2.00. When the molar ratio is within the above range, the pH of the aqueous phase of the mixed liquid will readily be adjusted to be within the above range.

The flow rate ratio of the first aqueous solution and the second aqueous solution to be mixed into the mixed aqueous solution, to be introduced to the reactor 20, is adjusted so that the total molar ratio of the basic alkali metal compound to the hydrogen peroxide in the respective solutions (total amount of basic alkali metal compound / hydrogen peroxide) is preferably within a range of from 0.5 to 1.5, more preferably from 0.6 to 1.3, particularly preferably from 0.65 to 1.2. When the molar ratio is within the above range, the yield can be maintained high without excessively using the raw materials.

The reaction temperature when the perfluoroacyl halide and hydrogen peroxide are reacted in the mixed liquid is preferably from -30 to 50°C, more preferably from -30 to 45°C, further preferably from -25 to 40°C, particularly preferably from -25 to 30°C. When it is at least the lower limit, the reaction time can be shortened, and in a case where the basic alkali metal is contained in the reaction system, solubility of the metal salt formed as a by-product by the reaction with the perfluoroacyl halide will be higher, whereby drawbacks such as clogging with the metal salt are less likely to occur, and thus the concentration of the raw materials can be increased, and more effective production will be possible. When it is at most the upper limit, decomposition reaction of the formed perfluoroacyl peroxide can be suppressed, thus improving the yield of the perfluoroacyl peroxide.

The reaction temperature corresponds to the liquid temperature of the mixed liquid when the compounds in the mixed liquid are reacted.

The retention time is preferably from 0.1 second to 5 hours, in view of excellent productivity.

In the method for producing a perfluoroacyl peroxide of the present invention, the recovered organic phase containing the perfluoroacyl peroxide may be purified. By purifying the recovered organic phase, the yield of the perfluoroacyl peroxide can further be improved.

For purification, operations such as distillation, dehydration, filtration, washing and recrystallization may be mentioned, and such operations may be conducted in combination.

By distillation, the organic solvent and the like contained in the organic phase can be removed. The distillation may be conducted e.g. by a known distillation apparatus.

By dehydration, moisture contained in the organic phase can be removed. Dehydration may be conducted using a dehydrating material such as a molecular sieve.

By filtration, solid matters and the like formed by the reaction can be removed. Filtration may be conducted by using e.g. a filter.

By washing, unreacted raw materials (for example the perfluoroacyl halide), solid matters formed by the reaction and the like which may be contained in the organic phase can be removed. As washing, a method of mixing deionized water and the organic phase may be mentioned.

By recrystallization, the organic solvent and the like contained in the organic phase can be removed. Recrystallization may be conducted using e.g. a known distillation apparatus.

The perfluoroacyl peroxide obtained by the method for producing a perfluoroacyl peroxide of the present invention is preferably a compound represented by the following formula (2):

$$\{R(C=O)O\}_2 \qquad (2)$$

wherein R is a group represented by $G(CF_2)_v[CF(CF_3)CF_2]_w[OCF(CF_3)CF_2]y[OCF(CF_3)]_z$-. G, v, w, y, z and v+w in R are respectively the same as G, v, w, y, z and v+w in the formula (1).

[Method for producing fluoroalkyl iodide]

In production of the fluoroalkyl iodide of the present invention, chain elongation that is telomerization by addition of the taxogen to the short chain $R^{fl}$ as the telogen, is utilized. As the taxogen, tetrafluoroethylene (hereinafter sometimes referred to as TFE) is used. Accordingly, the fluoroalkyl iodide of the telomer is obtained as $R^f(CF_2CF_2)_nI$ (n is the degree of polymerization).

Specifically, the method for producing a fluoroalkyl iodide of the present invention is a method of reacting a fluoroalkyl iodide represented by the following formula (3) and tetrafluoroethylene in the presence of the above obtained perfluoroacyl peroxide to obtain a fluoroalkyl iodide represented by the following formula (4):

$$R^fI \qquad (3)$$

$$R^fCF_2CF_2I \qquad (4)$$

wherein $R^f$ is a $C_{1-4}$ fluoroalkyl group.

The method for producing a fluoroalkyl iodide of the present invention is the same as WO2011/152499 except that the above obtained perfluoroacyl peroxide is used as a radical initiator.

In the method for producing a fluoroalkyl iodide of the present invention, the above obtained perfluoroacyl peroxide is used. The organic solvent which remains unremoved at the time of production of the perfluoroacyl peroxide may sometimes be used for production of the fluoroalkyl iodide together with the perfluoroacyl peroxide. Further, in view of handling efficiency of the perfluoroacyl peroxide, the organic phase containing the perfluoroacyl peroxide and the organic solvent may be used as it is, or the content of the perfluoroacyl peroxide may be adjusted with the same organic solvent as the organic solvent contained in the organic phase and a composition containing the perfluoroacyl peroxide and the organic solvent is used, for producing the fluoroalkyl iodide.

In such a case, usually, removal of the organic solvent is necessary at the time of production of the fluoroalkyl iodide. Whereas the perfluoroalkyl iodide ($C_2F_5I$, $C_4F_9I$, $C_6F_{13}I$) used as the organic solvent for production of the above perfluoroacyl peroxide is the same as the intermediate or the desired product obtained by the method for producing a fluoroalkyl iodide of the present invention, and thus such an advantage is obtained that removal of the organic solvent is unnecessary.

EXAMPLES

Now, the present invention will be described in further detail with reference to Examples. Ex. 1-1 to 1-3 and Ex. 2-1 to 2-2 are Examples of the present invention, and Ex. 1-4 to 1-5 and Ex. 2-3 are Comparative Examples. However, it should be understood that the present invention is not limited to such specific Examples. The amounts of the components in the after-described Tables are based on mass.

[Degree of conversion, yield]

The liquid obtained by the present reaction was subjected to composition analysis by concentration titration and gas chromatography to determine the degree of conversion of $C_2F_5COCl$, $(C_2F_5COO)_2$ selectivity and $(C_2F_5COO)_2$ yield, as follows.

<Concentration titration>

Into an Erlenmeyer flask having an internal capacity of 100 ml, 25 ml of acetic acid and 10 ml of a saturated aqueous potassium iodide solution were put in this order, and about 1.0 g of a sample was accurately weighed and added to the flask. The flask was plugged and the content was mixed and reacted at dark place for 10 minutes. Titration with a 0.1 mol/L aqueous sodium thiosulfate solution was conducted until the color of iodine disappeared. The same operation was conducted except that no sample was added (blank measurement). The concentration (mass%) of the product contained in the sample was calculated in accordance with the following formula.

$$\text{Concentration (mass\%) of product} = \{(V-Vb) \times Mw\} / (200 \times Sa)$$

V: volume (ml) of 0.1 mol/L aqueous sodium thiosulfate solution required for sample titration.
Vb: volume (ml) of 0.1 mol/L aqueous sodium thiosulfate solution required for blank measurement.
Mw: molecular weight 326 of product
Sa: mass (g) of sample

<Degree of conversion of $C_2F_5COCl$>

The degree of conversion of ($C_2F_5COCl$) was obtained from the results of the composition analysis in accordance with the following formula.

Degree of conversion of ($C_2F_5COCl$) (%) = {1-[($C_2F_5COCl$ concentration (mass%) at reactor outlet × (amount (g) of organic layer recovered in organic phase recovery tank))] / [($C_2F_5COCl$ concentration (mass%) at reactor inlet) × (amount (g) of organic solvent solution supplied to reactor)]} × 100

<($C_2F_5COO$)$_2$ selectivity>

The ($C_2F_5COO$)$_2$ selectivity was obtained from the results of composition analysis in accordance with the following formula.

$$\text{($C_2F_5COO$)}_2 \text{ selectivity (\%)} = \{\text{amount (mol) of ($C_2F_5COO$)}_2 \text{ formed} \times 2 / \text{amount (mol) of $C_2F_5COCl$ consumed}\} \times 100$$

<($C_2F_5COO$)$_2$ yield>

The ($C_2F_5COO$)$_2$ yield was obtained in accordance with the following formula. ($C_2F_5COO$)$_2$ yield (%) = (degree of conversion of $C_2F_5COCl$) × (($C_2F_5COO$)$_2$ selectivity)

[pH of aqueous phase of mixed liquid]

Using the aqueous phase (waste water) recovered in production of ($C_2F_5COO$)$_2$, pH of the aqueous phase at 25±2°C was measured by a pH meter (D-52, manufactured by HORIBA Ltd.).

[Solid matters in aqueous phase of mixed liquid]

Formation of solid matters in the aqueous phase (waste water) recovered in production of ($C_2F_5COO$)$_2$ was visually confirmed. The evaluation standards are as follows.

○: No solid matter observed
△: A small amount of solid matters formed; no precipitation observed in piping after the test but precipitation observed in crude liquid.
×: A large amount of solid matters formed; precipitation observed in pining after the test.

[Ex. 1-1]

As the organic solvent solution, a solution containing a perfluoroacyl halide ($C_2F_5COCl$) and an organic solvent ($C_4F_9I$) (perfluoroacyl halide content: 43.7 mass%) was prepared. As the first aqueous solution, an aqueous hydrogen peroxide solution (hydrogen peroxide content: 21.5 mass%) was prepared. As the second aqueous solution, an aqueous KOH solution (KOH content: 17 mass%) was prepared.

Further, as the reaction apparatus, the reaction apparatus 1 shown in Fig. 1 was used. As the reactor 20, a resin tube (tubular reactor) the outer periphery of which was covered with a jacket was used. The resin tube had a wetted part made of PFA, had a static mixer in its inside and had an inner diameter of 3 mm and a length of 1.5 m. ASAHIKLIN AC-6000 (trade name, manufactured by AGC Inc.) was used as a coolant for cooling the jacket to control the jacket temperature to be -20±2°C. The reaction temperature was -22 to 9.4°C.

First, the organic solvent solution stored in the storage tank 10A, the first aqueous solution stored in the storage tank 10B and the second aqueous solution stored in the storage tank 10C were introduced into the reactor 20 via the supply tube 12A to the supply tube 12C at flow rates as identified in Table 1. The flow rate of the organic solvent solution is the total of the flow rate of $C_2F_5COCl$ and the flow rate of $C_4F_9I$ in Table 1.

The respective solutions were mixed in the reactor 20 to obtain a mixed liquid containing the organic solvent solution, the first aqueous solution and the second aqueous solution.

Then, the mixed liquid after the reaction was discharged from the reactor 20 and introduced into the two phase separation tank 30 via the supply tube 22.

And, the two liquids separated in the two phase separation tank 30, that is the organic phase and the aqueous phase were recovered in the organic phase recovery tank 40A and in the aqueous phase recovery tank 40B respectively via the supply tube 32A and the supply tube 32B.

The organic phase contained $(C_2F_5COO)_2$ as the product and $C_4F_9I$ as the organic solvent.

[Ex. 1-2 to 1-5]

$(C_2F_5COO)_2$ was produced in the same manner as in Ex. 1-1 except that as the organic solvent solution, a solution containing a perfluoroacyl halide ($C_2F_5COCl$) and an organic solvent ($C_4F_9I$) (perfluoroacyl halide content: 43.9 mass%) was used, and the flow rates of the solutions were adjusted as identified in Table 1.

[Table 1]

| | | | Ex. 1-1 | Ex. 1-2 | Ex. 1-3 | Ex. 1-4 | Ex. 1-5 |
|---|---|---|---|---|---|---|---|
| Material of wetted part of reactor | | | PFA | PFA | PFA | PFA | PFA |
| Flow rate | $C_2F_5COCl$ | g/min | 6.91 | 6.90 | 6.82 | 8.18 | 9.49 |
| | $C_4F_9I$ | g/min | 8.90 | 8.80 | 8.72 | 10.44 | 12.14 |
| | Aqueous $H_2O_2$ solution | g/min | 8.25 | 8.30 | 7.66 | 9.24 | 10.81 |
| | Aqueous KOH solution | g/min | 16.32 | 16.16 | 14.26 | 16.94 | 20.03 |
| $KOH/C_2F_5COCl$ | | mol/mol | 1.31 | 1.30 | 1.16 | 1.15 | 1.17 |
| $KOH/C_4F_9I$ | | mol/mol | 1.92 | 1.92 | 1.71 | 1.70 | 1.73 |
| $H_2O_2/C_2F_5COCl$ | | mol/mol | 1.38 | 1.39 | 1.30 | 1.30 | 1.31 |
| $H_2O_2/C_4F9I$ | | mol/mol | 2.03 | 2.06 | 1.92 | 1.94 | 1.95 |
| $KOH/H_2O_2$ | | mol/mol | 0.95 | 0.93 | 0.89 | 0.88 | 0.89 |
| Degree of conversion of $C_2F_5COCl$ | | % | 94.9 | 97.4 | 95.6 | 93.7 | 90.8 |
| $(C_2F_5COO)_2$ yield | | % | 74.3 | 80.8 | 85.6 | 80.9 | 70.7 |
| pH of aqueous phase of mixed liquid | | - | 8.1 | 7.8 | 7.5 | 6.7 | 5.5 |
| Solid matters in aqueous phase of mixed liquid | | - | ○ | ○ | ○ | △ | × |

[Ex. 2-1]

A perfluoroacyl halide ($C_2F_5COCl$) and an organic solvent ($C_4F_9I$) were prepared. As the first aqueous solution, an aqueous hydrogen peroxide solution (hydrogen peroxide content: 35.0 mass%) was prepared. As the second aqueous solution, an aqueous KOH solution (KOH content: 15.0 mass%) was prepared.

Further, as the reaction apparatus, the reaction apparatus 100 as shown in Fig. 2 was used. As the reactor 20, a tube (tubular reactor) made of silicon carbide, the outer periphery of which was covered with a jacket was used. The tube had a static mixer in its inside, and had an inner diameter of 11 mm and a length of 2.0 m.

ASAHIKLIN AC-6000 (manufactured by AGC Inc.) was used as a coolant for cooling the jacket to adjust the jacket temperature to be -10±2°C. The reaction temperature was -12 to 7.9°C.

First, the perfluoroacyl halide was introduced into the reactor 20 via the supply tube 112A, the organic solvent via the supply tube 113A, the first aqueous solution stored in the storage tank 10B via the supply tube 12B, and the second aqueous solution stored in the storage tank 10C via the supply tube 12C, at the flow rates as identified in Table 2. The perfluoroacyl halide and the organic solvent were mixed before being introduced to the reactor 20 and introduced to the reactor 20 as an organic solvent solution containing the perfluoroacyl halide and the organic solvent.

The respective solutions were mixed in the reactor 20 to obtain a mixed liquid containing the organic solvent solution, the first aqueous solution and the second aqueous solution.

Then, the mixed liquid after the reaction was discharged from the reactor 20 and introduced to the two phase separation tank 30 via the supply tube 22.

And, the two liquids separated in the two phase separation tank 30, that is the organic phase and the aqueous phase were recovered in the organic phase recovery tank 40A and in the aqueous phase recovery tank 40B respectively via the supply tube 32A and the supply tube 32B.

The organic phase contained $(C_2F_5COO)_2$ as the product and $C_4F_9I$ as the organic solvent.

[Ex. 2-2]

$(C_2F_5COO)_2$ was produced in the same manner as in Ex. 2-1 except that a reactor having a wetted part made of PFA was used and the flow rates of the solutions were adjusted as identified in Table 2. The reaction temperature was -12 to 37.9°C.

[Ex. 2-3]

$(C_2F_5COO)_2$ was produced in the same manner as in Ex. 2-1 except that the flow rates of the solutions were adjusted as identified in Table 2. The reaction temperature was -12 to 9.5°C.

[Table 2]

|  |  |  | Ex. 2-1 | Ex. 2-2 | Ex. 2-3 |
|---|---|---|---|---|---|
| Material of wetted part of reactor |  |  | SiC | PFA | SiC |
| Flow rate | $C_2F_5COCl$ | g/min | 33.3 | 40.0 | 76.7 |
|  | $C_4F_9I$ | g/min | 86.7 | 84.8 | 86.7 |
|  | Aqueous $H_2O_2$ solution | g/min | 36.7 | 32.7 | 43.3 |
|  | Aqueous KOH solution | g/min | 110 | 115.9 | 176.7 |
| $KOH/C_2F_5COCl$ |  | mol/mol | 1.61 | 1.41 | 1.12 |
| $KOH/C_4F_9I$ |  | mol/mol | 1.17 | 1.26 | 1.89 |
| $H_2O_2/C_2F_5COCl$ |  | mol/mol | 2.07 | 1.54 | 1.06 |
| $H_2O_2/C_4F_9I$ |  | mol/mol | 1.51 | 1.37 | 1.78 |
| $KOH/H_2O_2$ |  | mol/mol | 0.78 | 0.92 | 1.06 |
| Degree of conversion of $C_2F_5COCl$ |  | % | 99.3 | 98.4 | 98.9 |
| $(C_2F_5COO)_2$ yield |  | % | 58.5 | 48.0 | 74.6 |
| pH of aqueous phase of mixed liquid |  | - | 12.1 | 7.5 | 5.3 |
| Solid matters in aqueous phase of mixed liquid |  | - | ○ | ○ | × |

It was confirmed that formation of solid matters can be suppressed according to the production method in Ex. 1-1 to 1-3 and Ex. 2-1 to 2-2 in which the pH of the aqueous phase of the mixed liquid is within a range of from 7 to 14, as shown in Tables 1 and 2, as compared with the prosecution method in Ex. 1-4 to 1-5 and Ex. 2-3 in which the pH of the aqueous phase of the mixed liquid is out of the above range.

Further, by comparison between Ex. 2-1 and Ex. 2-2, excellent yield due to high heat dissipation effect was confirmed by the production method in Ex. 2-1 in which the wetted part of the reactor was made of silicon carbide.

The entire disclosure of Japanese Patent Application No. 2019-130510 filed on July 12, 2019 including specification, claims, summary and drawings is incorporated herein by reference in its entirety.

REFERENCE SYMBOLS

1, 100: Reaction apparatus
10A, 10B, 10C: Storage tank
12A, 12B, 12C, 22, 32A, 32B, 112A, 113A: Supply tube
20: Reactor
30: Two phase separation tank
40A: Organic phase recovery tank
40B: Aqueous phase recovery tank

**Claims**

1.   A method for producing a perfluoroacyl peroxide, which comprises:

mixing an organic solvent solution containing a perfluoroacyl halide and an organic solvent which is at least one perfluoroalkyl iodide selected from the group consisting of $C_2F_5I$, $C_4F_9I$ and $C_6F_{13}I$,
a first aqueous solution containing hydrogen peroxide, and
a second aqueous solution containing a basic alkali metal compound,
to obtain a mixed liquid, and
reacting the perfluoroacyl halide and the hydrogen peroxide in the mixed liquid to produce a perfluoroacyl peroxide,
wherein the pH of the aqueous phase of the mixed liquid is adjusted to be from 7.0 to 14.0.

2.   The method for producing a perfluoroacyl peroxide according to Claim 1, wherein the first aqueous solution and the second aqueous solution are mixed to obtain a mixed aqueous solution, and the mixed aqueous solution and the organic solvent solution are mixed in a reactor.

3.   The method for producing a perfluoroacyl peroxide according to Claim 2, wherein a wetted part of the reactor is made of silicon carbide.

4.   The method for producing a perfluoroacyl peroxide according to any one of Claims 1 to 3, wherein the perfluoroacyl halide is a compound represented by the following formula (1):

$$G(CF_2)_v[CF(CF_3)CF_2]_w[OCF(CF_3)CF_2]_y[OCF(CF_3)]_z\text{-}(C=O)X \qquad (1)$$

wherein G is a fluorine atom or a pentafluorophenoxy group, X is a halogen atom, v is an integer of from 0 to 10, w is 0 or 1, y is an integer of from 0 to 7, and z is 0 or 1, provided that $v+w\geqq1$ is satisfied.

5.   The method for producing a perfluoroacyl peroxide according to any one of Claims 1 to 4, wherein the first aqueous solution is an aqueous hydrogen peroxide solution.

6.   The method for producing a perfluoroacyl peroxide according to any one of Claims 1 to 4, wherein the first aqueous solution contains a metal peroxide.

7.   The method for producing a perfluoroacyl peroxide according to any one of Claims 1 to 6, wherein the basic alkali metal compound is potassium hydroxide.

8.   The method for producing a perfluoroacyl peroxide according to any one of Claims 1 to 7, wherein the content of the perfluoroacyl halide in the organic solvent solution to the total mass of the organic solvent solution is from 1 to 80 mass%.

9.   The method for producing a perfluoroacyl peroxide according to any one of Claims 1 to 8, wherein the perfluoroacyl halide and the hydrogen peroxide are reacted at from -30 to 50°C.

10. The method for producing a perfluoroacyl peroxide according to any one of Claims 1 to 9, wherein the organic solvent solution and the first aqueous solution are mixed so that the molar ratio of the hydrogen peroxide to the perfluoroacyl halide is from 1.0 to 3.0.

11. The method for producing a perfluoroacyl peroxide according to any one of Claims 1 to 10, wherein the organic solvent solution and the second aqueous solution are mixed so that the molar ratio of the basic alkali metal compound to the perfluoroacyl halide is from 1.0 to 2.0.

12. The method for producing a perfluoroacyl peroxide according to any one of Claims 1 to 11, wherein the organic solvent solution and the second aqueous solution are mixed so that the molar ratio of the basic alkali metal compound to the perfluoroalkyl iodide is from 1.0 to 2.2.

13. The method for producing a perfluoroacyl peroxide according to any one of Claims 1 to 12, wherein the first aqueous solution and the second aqueous solution are mixed so that the molar ratio of the basic alkali metal compound to the hydrogen peroxide is from 0.5 to 1.5.

14. A method for producing a fluoroalkyl iodide, which comprises reacting a fluoroalkyl iodide represented by the following formula (3) and tetrafluoroethylene in the presence of a perfluoroacyl peroxide obtained by the method for producing a perfluoroacyl peroxide as defined in any one of Claims 1 to 13, to obtain a fluoroalkyl iodide represented by the following formula (4):

$$R^f I \qquad (3)$$

$$R^f CF_2 CF_2 I \qquad (4)$$

wherein $R^f$ is a $C_{1-4}$ fluoroalkyl group.

Fig. 1

Fig. 2

## INTERNATIONAL SEARCH REPORT

| International application No. |
| --- |
| PCT/JP2020/026079 |

**A. CLASSIFICATION OF SUBJECT MATTER**
Int. Cl. C07C407/00(2006.01)i, C07C17/278(2006.01)i, C07C19/16(2006.01)i, C07C409/34(2006.01)i
FI: C07C407/00 ZAB, C07C17/278, C07C19/16, C07C409/34
According to International Patent Classification (IPC) or to both national classification and IPC

**B. FIELDS SEARCHED**

Minimum documentation searched (classification system followed by classification symbols)
Int. Cl. C07C407/00-409/44, C07C17/00-19/16

Documentation searched other than minimum documentation to the extent that such documents are included in the fields searched
Published examined utility model applications of Japan    1922-1996
Published unexamined utility model applications of Japan  1971-2020
Registered utility model specifications of Japan          1996-2020
Published registered utility model applications of Japan  1994-2020

Electronic data base consulted during the international search (name of data base and, where practicable, search terms used)
CAplus (STN); CASREACT (STN); REGISTRY (STN)

**C. DOCUMENTS CONSIDERED TO BE RELEVANT**

| Category* | Citation of document, with indication, where appropriate, of the relevant passages | Relevant to claim No. |
| --- | --- | --- |
| A | JP 5-97797 A (NOF CORP.) 20 April 1993, claims, examples | 1-14 |
| A | JP 6-172303 A (NOF CORP.) 21 June 1994, claims, examples | 1-14 |
| A | JP 11-49749 A (NOF CORP.) 23 February 1999, claims, examples | 1-14 |
| A | JP 11-511464 A (E. I. DU PONT DE NEMOURS & CO.) 05 October 1999, claims, examples | 1-14 |
| A | JP 2000-7779 A (AUSIMONT S. P. A.) 11 January 2000, claims, examples | 1-14 |

☒ Further documents are listed in the continuation of Box C.  ☒ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| 09.09.2020 | 24.09.2020 |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.

PCT/JP2020/026079

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2010-18532 A (ASAHI GLASS CO., LTD.) 28 January 2010, claims, examples | 1-14 |

Form PCT/ISA/210 (continuation of second sheet) (January 2015)

INTERNATIONAL SEARCH REPORT

Information on patent family members

International application No.

PCT/JP2020/026079

| Patent Documents referred to in the Report | Publication Date | Patent Family | Publication Date |
|---|---|---|---|
| JP 5-97797 A | 20.04.1993 | (Family: none) | |
| JP 6-172303 A | 21.06.1994 | (Family: none) | |
| JP 11-49749 A | 23.02.1999 | (Family: none) | |
| JP 11-511464 A | 05.10.1999 | US 5831131 A claims, examples CA 2230606 A1 DE 69618646 T2 EP 0847387 A1 US 5962746 A WO 97/08142 A1 | |
| JP 2000-7779 A | 11.01.2000 | US 6187894 B1 claims, examples DE 69907323 T2 EP 0970984 A2 IT MI981328 A1 | |
| JP 2010-18532 A | 28.01.2010 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010117029 A
- WO 2011152499 A
- JP 2019130510 A